(19) Europäisches Patentamt European Patent Office Office européen des brevets

(11) **EP 4 484 579 A1**

(12) **EUROPEAN PATENT APPLICATION**

(43) Date of publication:
01.01.2025 Bulletin 2025/01

(21) Application number: 23182883.1

(22) Date of filing: 30.06.2023

(51) International Patent Classification (IPC):
*C12Q 1/689* (2018.01)

(52) Cooperative Patent Classification (CPC):
**C12Q 1/689;** C12Q 2600/124

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Adisseo France S.A.S.**
**92160 Antony (FR)**

(72) Inventors:
• **Vieco Saiz, Nuria**
**69007 Lyon (FR)**
• **Consuegra Bonilla, Jessika**
**69630 Chaponost (FR)**

(74) Representative: **Germain Maureau**
**12, rue Boileau**
**69006 Lyon (FR)**

(54) **BIOMARKER OF THE STATUS OF THE MICROBIOTA OF A FARM ANIMAL AND USES THEREOF**

(57) The present invention relates to a use of one or more ratios of quantities of bacteria in the microbiota of a farm animal, in animal feed, said ratio(s) being selected from *Firmicutes / Proteobacteria; Clostridium* Clusters IV and XIVa / *Enterobacteriaceae; Ruminococcaceae* and *Lachnospiraceae / Enterobacteriaceae;* butyrate-producing bacteria / *Firmicutes*; aerobic bacteria / anaerobic bacteria. It/They may be used as marker(s) to determine the status of the intestinal microbiota of a farm animal or for selecting an extract of a satisfactory intestinal microbiota of a farm animal, intended to maintain or to improve the intestinal microbiota of a different farm animal in need thereof.

EP 4 484 579 A1

**Description**

The present invention relates to the field of livestock animals and more particularly pertains to an alternative way for improving their health. To mitigate the problems of health of farm animals is a major stake for any stockbreeder, in that it is definitively linked to high performance in terms of production and meat quality, and from an ethical point of view, to a better animal welfare.

Animal's growth performance is usually carried out by measuring zootechnical parameters such as body weight (BW), body weight gain (BWG), feed intake (FI) and feed conversion ratio (FCR). Regarding the assessment of animal's health, parameters are measured on the animals or their environment. Commonly used parameters are based on the detection of plasma proteins in the luminal fluid, of short chain fatty acids (SCFA) production in the gut microbiota, of lipopolysaccharides (LPS) in serum, of bacteria passing through the intestinal epithelium (bacterial translocation), etc. (Ducatelle, R.; Goossens, E.; De Meyer, F.; Eeckhaut, V.; Antonissen, G.; Haesebrouck, F.; van Immerseel, F. (2018) Biomarkers for monitoring intestinal health in poultry. Present status and future perspectives. In : Veterinary research, vol. 49, n° 1, p. 43). Regarding external parameters it is usually measured by litter and pododermatitis scoring (Eichner, G.; Vieira, S. L.; Torres, C. A.; Coneglian, J.L.B.; Freitas, D. M.; Oyarzabal, O. A. (2007) Litter Moisture and Footpad Dermatitis as Affected by Diets Formulated on an All-Vegetable Basis or Having the Inclusion of Poultry By-Product. In : The Journal of Applied Poultry Research, vol. 16, n° 3, p. 344-350).

Gastrointestinal tract (GIT) health and more specifically intestinal health is a key factor for general health and well-being of animals and consequently on production.

In the context of livestock, animals face significant challenges, in particular due to the increase in farm size, ever faster growth, sensitive physiological stages, thus their performance can be significantly reduced. Resilience is the ability of animals to adapt in this challenging environment through greater resistance. More resilient animals will have more regular production and better welfare.

In accordance with the present invention, it was discovered that determined microbial ratios of intestinal microbiota can be used, on the one hand, as a biomarker of the status of this microbiota and, on the other hand, for selecting microbial inocula having applications such as for maintaining or improving the GIT health of farm animals or for developing resilience of young animals that will grow under farming conditions.

Thereby, the invention lies about the use, in animal feed, of one or more ratios of quantities of bacteria in the microbiota of a farm animal, said ratio(s) being selected from *Firmicutes*/*Proteobacteria; Clostridiu*m Clusters IV and XIVa / *Enterobacteriaceae; Ruminococcaceae* and *Lachnospiraceae* / *Enterobacteriaceae;* butyrate-producing bacteria / *Firmicutes;* aerobic bacteria / anaerobic bacteria,

In an aspect, the invention relates to the use of said ratio(s) as marker(s) to determine the status of the intestinal microbiota of a farm animal.

In a further aspect, the invention pertains to the use of said ratio(s) for selecting an extract of a satisfactory intestinal microbiota of a farm animal, said extract being intended to maintain or to improve the intestinal microbiota of a farm animal in need thereof, the latter being different from the farm animal from which said extract comes from.

In an embodiment, the invention aims at a biomarker of the status of the microbiota of a farm animal, said biomarker being selected from the following ratios of amounts of bacteria,

*Firmicutes* / *Proteobacteria,*
*Clostridium* Clusters IV and XIVa / *Enterobacteriaceae,*
*Ruminococcaceae* and *Lachnospiraceae* / *Enterobacteriaceae,*
butyrate producing bacteria / *Firmicutes,* and
aerobic bacteria / anaerobic bacteria.

This new biomarker directly reflects the intestinal health of a farm animal and may find many applications in the breeding business in view of its consequences on production.

Thus, in accordance with the present invention, it is provided a method for determining *in vitro* or ex *vivo* a status of the intestinal microbiota of a farm animal, said method comprising at least the following steps:

detecting and quantifying, in a biological sample of said farm animal, bacteria selected from the *Firmicutes*, *Proteobacteria, Clostridium* Clusters IV and XIVa, *Enterobacteria, Ruminococcaceae, Lachnospiraceae,* butyrate-producing bacteria, aerobic bacteria and anaerobic bacteria; and
calculating at least one ratio selected from

*Firmicutes* / *Proteobacteria,*
*Clostridium* Clusters IV and XIVa / *Enterobacteriaceae,*
*Ruminococcaceae* and *Lachnospiraceae* / *Enterobacteriaceae,*

butyrate producing bacteria / *Firmicutes*, and
aerobic bacteria / anaerobic bacteria.

In a further embodiment of the invention, it is provided a method for selecting a satisfactory extract of the intestinal microbiota of a farm animal, said method comprising the following steps:

detecting and quantifying, in a biological sample of said farm animal, the quantity of each of the bacteria selected from *Firmicutes*, bacteria producing butyrate, aerobic bacteria and anaerobic bacteria and calculating at least one ratio selected from butyrate-producing bacteria / *Firmicutes* (a1), aerobic bacteria / anaerobic bacteria (a1);
detecting and quantifying, in a biological sample of a different farm animal, the quantity of each of the bacteria selected from *Firmicutes*, the bacteria producing butyrate, aerobic bacteria and anaerobic bacteria and calculating at least one ratio selected from butyrate-producing bacteria / *Firmicutes* (a2), aerobic bacteria / anaerobic bacteria (b2);
comparing the calculated ratios (a1) and (b1) and/or (a2) and (b2); and
selecting said satisfactory extract from said comparison.

According to yet another embodiment of the invention, it is provided a non-therapeutic method for maintaining or improving the intestinal microbiota of a farm animal in need thereof, said method comprising the following steps:

selecting a satisfactory microbiota extract according to the method as above described, and
administrating to said animal, said satisfactory microbiota extract or a derivative thereof resulting from fermentation, said animal being different from the one from which the satisfactory extract or derivative thereof is selected.

A healthier intestinal environment would ensure intestinal health and thus favor animal resilience against challenges.

Before the invention is exposed, some terms that are employed in the present text are defined.

A microbial transfer (MT) is the process by which a microbial extract, also named microbial inoculum, from a selected animal, is administrated to an animal in need thereof. An intestinal microbial transfer (IMT) is equivalent to a fecal microbial transfer (FMT) but extends to other types of inocula than fecal matter such as cecal and ileal matters, and their fermentation products. In accordance with the present invention, such microbial transfer is made via the animal feed or drinking water.

A microbial inoculum is a population of microorganisms from a selected animal, that is given to an animal, said selected animal being considered as resilient in comparison with the animal to which the microbial inoculum is administrated, either because said animal is suffering dysbiosis or because it is young having a poor and/or immature microbiota. This population of microorganisms can be isolated from samples such as feces, intestinal contents (cecal or ileal) and/or derived from said samples, for example by their fermentation.

In the present text, a farm animal having a satisfactory intestinal microbiota is understood to be a farm animal which has an intestinal microbiota that has reached maturity; this usually occurs from 3 weeks-old for poultry. This characteristic which essentially depends on the age of the animal is a well-known concept from the skilled in the art; as illustration in the context of the invention, a farm animal having a satisfactory intestinal microbiota is a farm animal that is not in need of an extract of a satisfactory intestinal microbiota. The expressions "an extract of a satisfactory intestinal microbiota" and "a satisfactory extract of an intestinal microbiota" have the same meaning.

A farm animal in need of maintaining or improving its intestinal microbiota is an animal which resilience and resistance to improve capacity are enhanced upon challenge in order to reduce performance losses; in the gut, there are interactions between intestinal microorganisms and the host that can be mutually beneficial or detrimental and thus modulate health or disease status; those interactions are stronger at early age because the gut is immature, and the defense mechanisms are still developing; so such an animal may be a young animal having a microbiota which has not reached maturity ; in poultry, they are chicken 10 days-old or less.

By control or control value, it is encompassed a value which may be measured for an animal which is not challenged, and may be, in this case, a specific value or may belong to a value range; it also encompasses a value which may be measured for an animal which is challenged, but which ratio(s) is/are more satisfactory than the values resulting from the other challenged animals (also called resilient animal).

The present invention will be exposed and detailed below in reference to intestinal health and microbiota of poultry, but it extends to any other animals and in particular to any farm animals in view of the productions concerns; such farm animals are monogastric animals first, for example pigs.

In accordance with an embodiment of the invention, the method for determining *in vitro* or ex *vivo* status of the intestinal microbiota of a farm animal further comprises the following steps:

comparing the or each of the ratio(s) of said biological sample of said animal with a control value determined for the or each of said ratio(s), and
determining the status of said microbiota from said comparison.

Preferably, at least one ratio selected from *Firmicutes* / *Proteobacteria, Clostridium* Clusters IV and XIVa / *Enterobacteriaceae, Ruminococcaceae* and *Lachnospiraceae* / *Enterobacteriaceae,* butyrate-producing bacteria / *Firmicutes* is calculated, and
if the ratio or each of the calculated ratios is greater than or equal to the control value determined for the corresponding ratio, then the status of the microbiota is qualified as satisfactory, and if the ratio or each of the ratios is lower than the control value, then the status of the microbiota is qualified as unsatisfactory.

The above method may comprise calculating and optionally comparing with a control value, at least two, at least three, or at least four of said ratios, or the five ratios.

A biological sample is advantageously selected from cecal content, ileal content, fecal content and any fermentation derivatives thereof.

The abundance of bacteria is preferably measured by the relative abundances obtained from data resulting from the sequencing analysis by 16s rRNA sequencing, but it can in fact be measured by any other appropriate analysis which is well-known from the skilled in the art, and for example by any other metagenomics sequencing such as Whole Genome Shotgun (WGS ST Park and J. Kim. Trends in Next-Generation Sequencing and a New Era for Whole Genome Sequencing, Int. Neurourol J. 2016 Nov; 20(Suppl 2): S76-83 ), or by quantitative PCR (qPCR).

In a specific implementation of this method, it is determined the status of the microbiota of a chicken and the control value of any of the following ratios *Firmicutes* / *Proteobacteria; Clostridium* Clusters IV and XIVa / Enterobacteriaceae; *Ruminococcaceae* and *Lachnospiraceae* / *Enterobacteriaceae;* butyrate-producing bacteria / *Firmicutes*; aerobic bacteria / anaerobic bacteria is 60 and/or the control value of the aerobic bacteria / anaerobic bacteria ratio is measured in a chicken under farm conditions that is not or less challenged than those of the chicken which intestinal microbiota status is determined.

As mentioned above, in a further application of a biomarker of the invention, it is provided a method for selecting an extract of a satisfactory intestinal microbiota of a farm animal.

In a preferred implementation, this method may be carried out as follows:
if the butyrate-producing bacteria / *Firmicutes* ratio is the highest, and/or if the aerobic bacteria/anaerobic bacteria ratio is the lowest, among the values determined for all the samples tested, said extract is considered as satisfactory and is therefore selected.

In accordance with an embodiment of the invention, the non-therapeutic method for maintaining or improving the intestinal microbiota of a farm animal in need thereof, as described above, is intended for young farm animal, such as chicken, which intestinal microbiota is not still mature.

As an illustration, if the farm animal is a chick, it is administered an amount of $1-9.10^7$ CFU of said extract.

Said extract may be used immediately or after it has been stored in such conditions that it remains stable. In a preferred embodiment, the extract is fermented. Any fermentation may be carried out on said extract, provided that it helps promote the required ratios.

Another subject of the invention is a use of one or more ratios of quantities of bacteria in the microbiota of a farm animal, said ratio(s) being selected from *Firmicutes* / *Proteobacteria; Clostridium* Clusters IV and XIVa / *Enterobacteriaceae; Ruminococcaceae* and *Lachnospiraceae* / *Enterobacteriaceae;* butyrate-producing bacteria / *Firmicutes*; aerobic bacteria / anaerobic bacteria, as marker(s) to determine the status of the intestinal microbiota of a farm animal.

Yet another subject of the invention is a use of an extract of a satisfactory intestinal microbiota of a farm animal, as a feed additive for maintaining or improving the intestinal microbiota of a different farm animal in need thereof. Advantageously, said satisfactory extract is selected with the method for selecting an extract of a satisfactory intestinal microbiota of a farm animal as described above. In further preferred embodiment, said selected extract is then fermented.

The present invention is illustrated in the examples below from which its interest and benefic will arise.

At the end of these examples, the protocol below was followed.

Chickens were divided in groups, one group having a standard diet (STD) and each ot the other groups being administrated a treatment as defined below. The ceca of the chickens were extracted, the microbiota was analyzed by 16s sequencing and the ratios were calculated thanks to the taxonomy tables generated from Amplicon Sequence Variant (ASV).

The studies shown below are a sample of the different challenges that the animals may face:

nutritional problems such as the inclusion of rye and fat (Mild dysbiosis, MLD); or the addition of galactomannan (GAL);
challenges due to bacterial or parasitic infections such as severe dysbiosis regimen (same diet as MLD but with a supplementation of bacteria, SV), necrotic enteritis (NE).

In all of them it is seen how the expected trend is followed when the animals are challenged.

In the following examples, it is referred to the following figures:

**Figure** 1: Boxplots of the ratio 2.1 calculated in 4 studies, MLD, SV, GAL and NE, and compared to STD.

**Figure** 2: Boxplots of the ratio 2.3 calculated in 4 studies, MLD, SV, GAL and NE, and compared to STD.

**Figure** 3: Boxplots of the ratio 2.4 calculated in 4 studies, MLD, SV, GAL and NE, and compared to STD.

**Figure** 4: Boxplots of the ratio 2.15 calculated in 4 studies, MLD, SV, GAL and NE, and compared to STD.

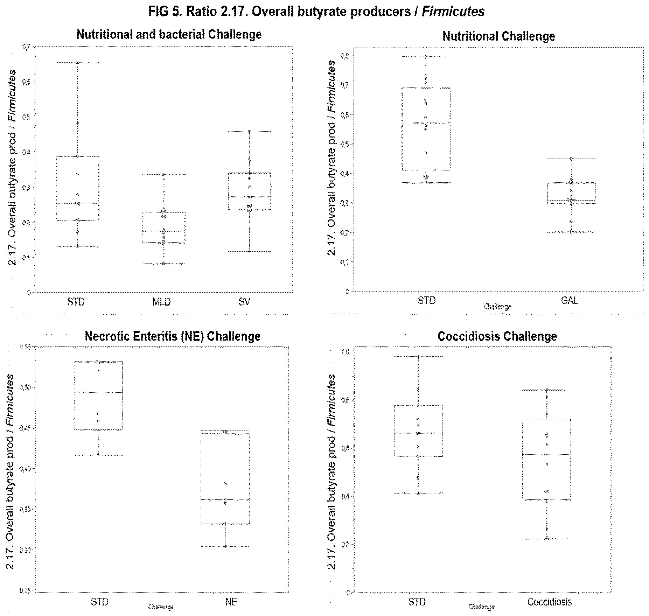

**Figure** 5: Boxplots of the ratio 2.17 calculated in 4 studies, MLD, SV, GAL and NE, and compared to STD.

**Figure** 6: Boxplots of the ratio Redox 1 calculated in 4 studies, MLD, SV, GAL and NE, and compared to STD.

**Figure** 7: Boxplots of the ratio Redox 2 (Ln) calculated in 4 studies, MLD, SV, GAL and NE, and compared to STD.

**Figure 8:** SCFA producers ratio and redox ratio of Inoculum A and Inoculum B.

**Figure 9:** Effects of inocula on body weight of 7-day old chickens (p-value: 0,007).

## Example 1 : Description of selected Ratios

### 1.1) Ratio 2.1: *Firmicutes* / *Proteobacteria*

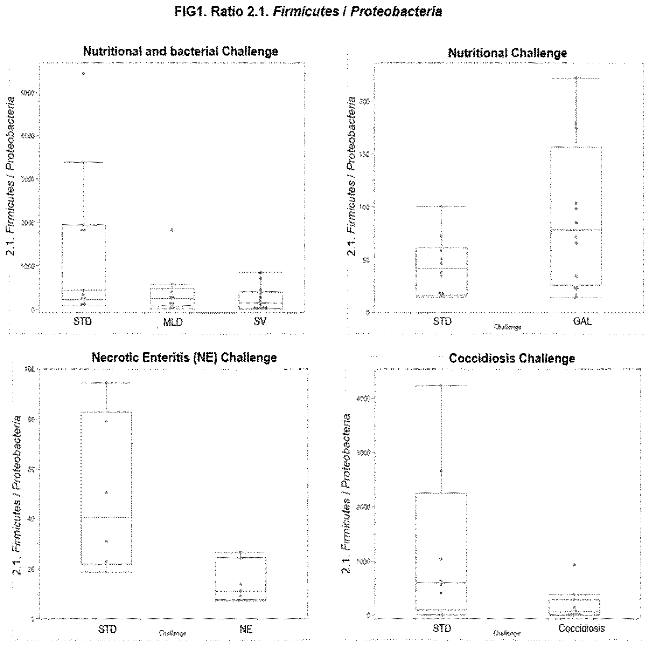

In 4 studies, the ratio varies in the expected way where the challenge was based on nutritional dysbiosis (fat, fiber, viscosity in diet, generally modification of the nutritional profile) and necrotic enteritis (Figure 1). It was considered as relevant to assess the gut health status since the confidence index was 60%. The values of the ratio differ from one study to the other being difficult to set up a reference value of "health/resilience".

### 1.2) Ratio 2.3: *Clostridium* Clusters IV and XIVa (CCs)/Enterobacteriaceae

*Clostridium* Clusters IV and XIVa (CCs) were set in the numerator because of their capabilities using large amounts of nutrients that cannot be digested by host and producing short-chain fatty acids (SCFAs) in high quantities, which play a noticeable role in intestinal homeostasis. They are able to attenuate inflammation because of their distinctive biological activities such as production of butyrate, secondary bile acids and indole propionic acid.

In standard conditions, when no challenge is induced, the ratio should be higher than with challenges, reflecting a bigger proportion of CCs than *Enterobacteriaceae,* or a decline of the *Enterobacteriaceae.*

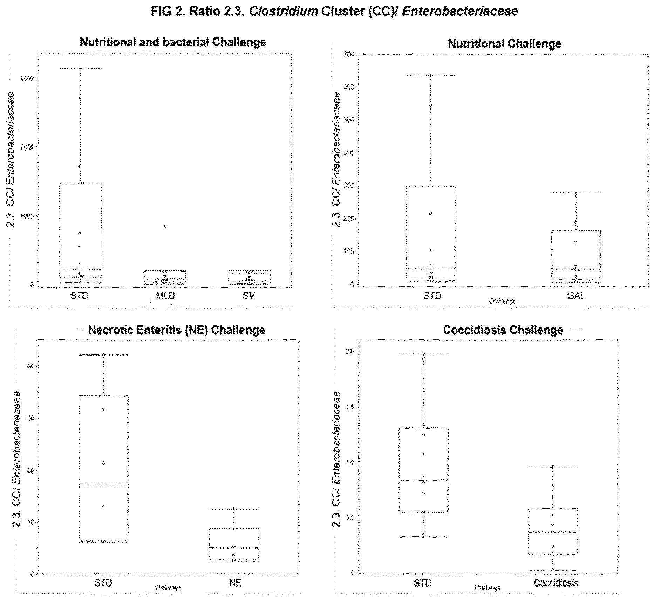

Ratio 2.3 varies as expected in all the studies but on dysbiosis group on day 20 for both dysbiosis mild (MLD) and severe (SD). The corresponding confidence index was 80% (Figure 2).

### 1.3) Ratio 2.4: *Ruminococcaceae* and *Lachnospiraceae* / *Enterobacteriaceae*

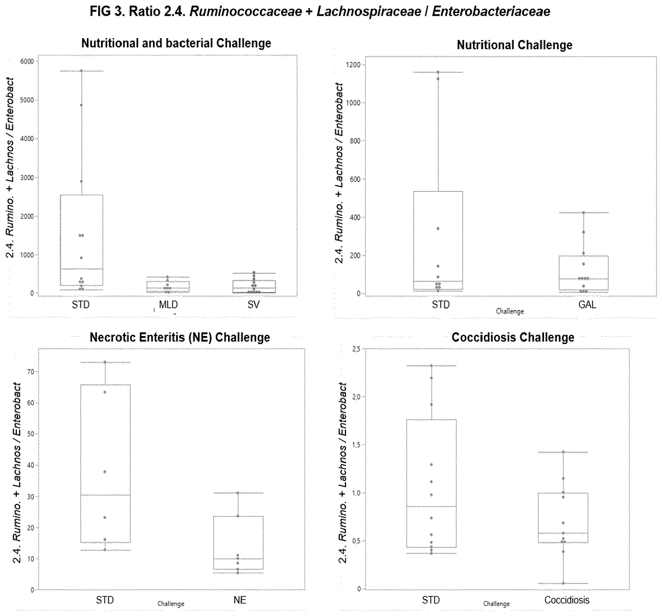

The major difference between the ratio 2.4 and 2.3 is the utilization of different taxa. When the ratio 2.3 were made to evaluate the amount of CCs in the microbiota, the ratio 2.4 allows to evaluate the proportion of two major SCFA producing families: *Lachnospiraceae* and *Ruminococcaceae,* and especially butyrate producers. The *Lachnospiraceae* family is a phylogenetically and morphologically heterogeneous taxon belonging to the clostridial cluster XIVa of the *phylum Firmicutes*; *Ruminococcaceae* is to Cluster IV. It is expected an increase of this ratio on the control group compared to the challenges, reflecting a bigger proportion of *Ruminococcaceae* and *Lachnospiraceae* than *Enterobacteriaceae,* or a decline of the *Enterobacteriaceae* (Figure 3). The trend is like the 2.3 due to *Clostridium* clusters from ratio 2.3 belongs to *Ruminococcaceae* and *Lachnospiraceae,* the differences between these 2 ratios is that in the families, there are more genera used in the ratio compared to the CCs. The ratio 2.4 presents a confidence of 80%.

### 1.4) Ratio 2.15: Butyrate producers / *Firmicutes*

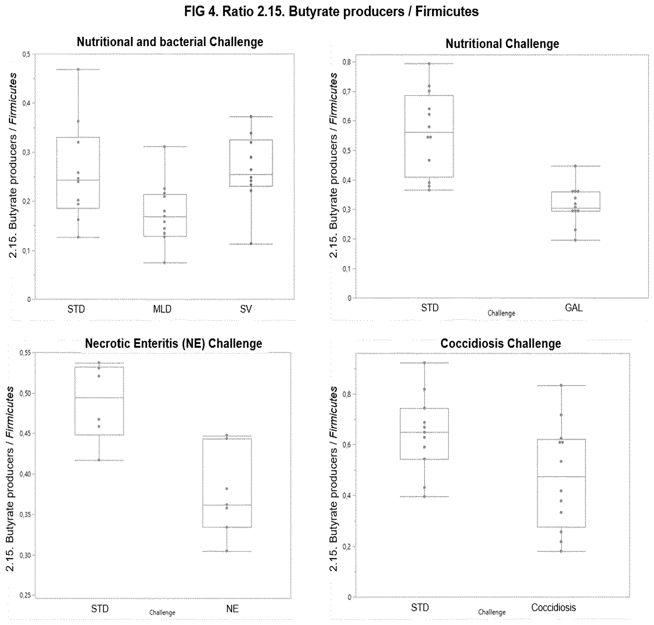

The ratio 2.15 assess the proportion of butyrate producers' genera in the phylum of *Firmicutes. Bacteroidetes* (gram-negative) and *Firmicutes* (gram-positive) are the most abundant phyla in the intestine, with members of the *Bacteroidetes* mainly producing acetate and propionate, while *Firmicutes* mostly produce butyrate in the human gut. Therefore, in good health condition (standard diet) the ratio will be higher than in challenges, turn into an augmentation of bacteria able to produce butyrate that has a beneficial effect on the colonocytes strengthening the epithelial barrier, reducing inflammation, and increasing the productions of mucins and antimicrobial peptides. Compared to the other ratios, the ratio 2.15 varies between 0 and 1 because it evaluates the proportion of several genera able to produce butyrate in an entire phylum. Figure 4 shows ratio 2.15 follows the expected trend in 4 studies where there are a dysbiosis created by fat in the diet (MLD) and necrotic enteritis challenge (NE). It leads to a confidence index of 60% (Figure 4).

1.5) Ratio 2.17: Butyrate producers (based on Vital, 2017 + those from ratio 2.15) / *Firmicutes*

Ratio 2.17 completes the list of butyrate producers suggested in ratio 2.15. (based on what Vital, M.; Karch, A.; Pieper, D. H. (2017) Colonic Butyrate-Producing Communities in Humans: an Overview Using Omics Data. In: mSystems, vol. 2, n° 6 described), adding 6 new taxa to the precedent ratio. This ratio is expected to be higher in standard conditions than in challenges, which means an augmentation of butyrate producing genera in standard condition. The arbitrary units for this ratio are delimited between 0 and 1. Slightly differences are remarked in comparison to ratio 2.15. However, it varies in the expected way in all the challenges. The confidence index for it is 60%.

1.6) Ratios Red/Ox

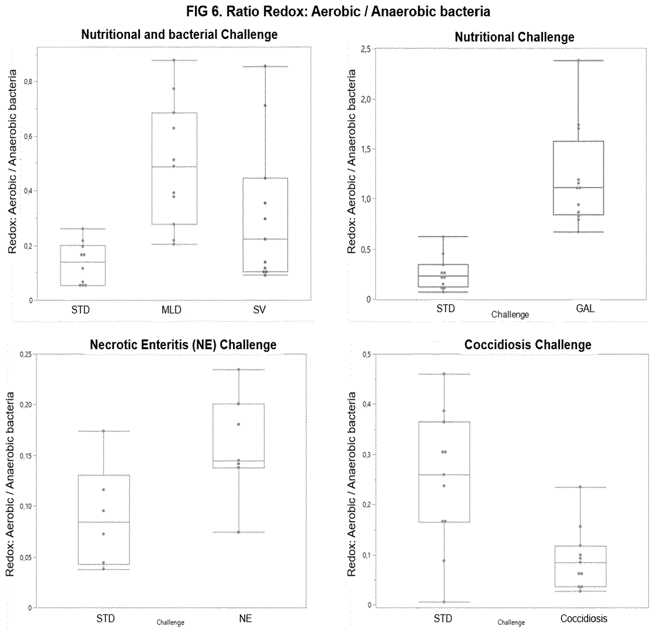

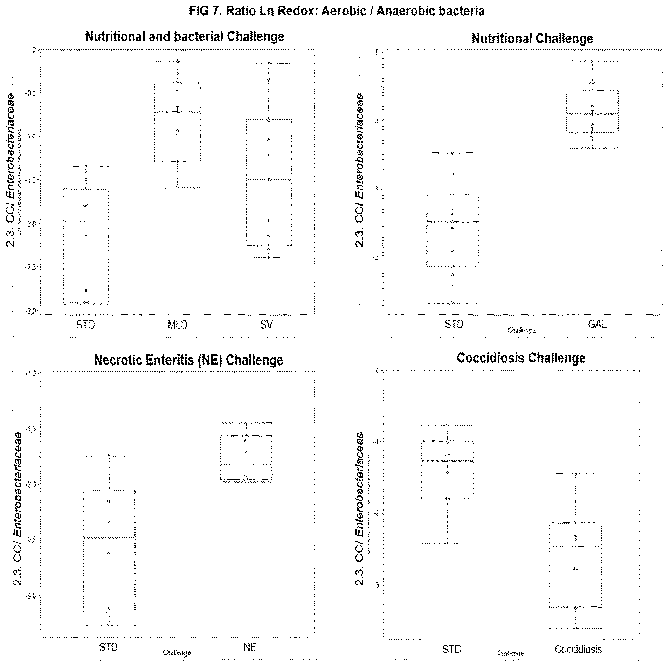

Ratio linked to redox activity and its Napierian logarithm were proposed. They are based on what Million, M.; Raoult, D. (2018) Linking gut redox to human microbiome. In : Human Microbiome Journal, vol. 10, p. 27-32 described. It divides the microbiota components in two groups aerobic and anaerobic bacteria. In human, those ratios link the Red/Ox potential of the intestinal tract with the microbiota and an inflammatory state in the colon. High concentration of aerobic bacteria was linked to an oxidant stress by an augmentation of the reactive oxygen species, aerobic bacteria proliferate preferentially in the intestinal microbiota to the detriment of anaerobic bacteria. The ratios were based on an exhaustive and precise list of specific aerobic or anaerobic species. By lack of data on species and in a concern of homogeneity, ratios were re designed and abundances relatives of species were replaced by relative abundances of genera (cf. above-cited Million et Raoult 2018, Annexe 4). Figure 6 shows how ratio Redox 1 varies in the expected way in the Trial 1 at D13, 28 and 35, and the trials 3, 4 and 5. The ratio has a confidence index equal to 66%. Redox ratio 2 (LnRatio) follows the expected trend on the same studies than the Redox ratio 1one trial, decreasing the confidence index to 55% (Figure 7).

**Example 2: Selection of ratios**

The microbial inocula were selected according with their profile on the following microbial ratios:

2.1) Ratio on Short Chain Fatty Acids (SCFA) producers against enterobacteria

$$SCFA\ producers\ ratio = \frac{Ruminococcacceae + Lachnospiraceae}{Enterobacteriaceae}$$

SCFA are molecules that improve the gut health through different mechanisms such as providing energy to intestinal cells, maintenance of intestinal barrier integrity, mucus production, and protection against inflammation. This ratio contains *Ruminococcaceae* and *Lachnospiraceae* families, having a SCFA-producing capacity, and members of the *Enterobacteriaceae* family, such as E. *coli* or *Salmonella,* which cause infections in the gastrointestinal tract.

2.2) Ratio redox

$$Redox\ ratio = \frac{Aerobes}{Anaerobes}$$

An inoculum containing a low redox ratio could lead to a better resilience facing challenges. This ratio is based on the optimal environment for cecal bacteria to correctly perform their activity, usually anaerobic. Many pathogens are oxygen tolerant, measuring those bacteria consider aerobes or anaerobes could reflect the health status of the gut.
To calculate those ratios, samples were subjected to 16s rRNA sequencing and ratios were calculated using the relative abundances tables obtained from the sequencing.
The following inocula were selected:

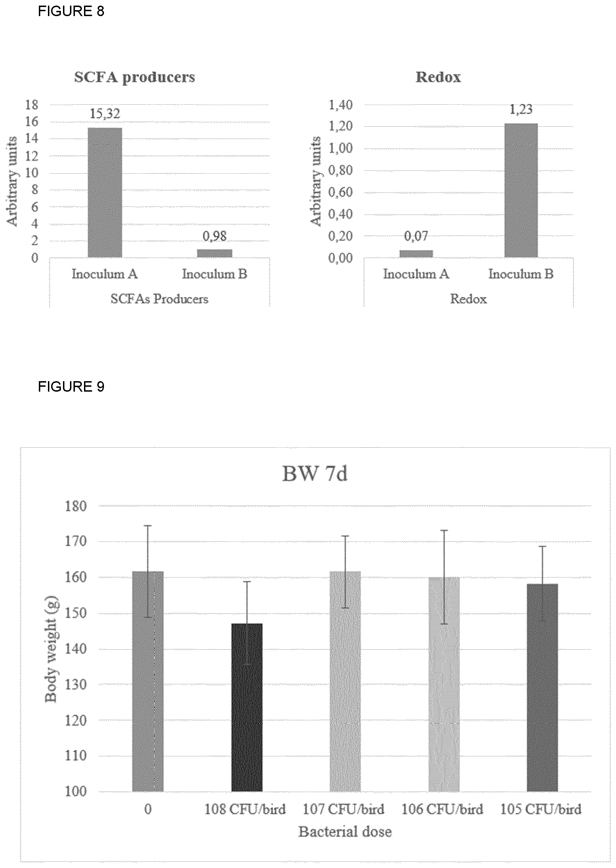

Inoculum A: Inoculum with a high ratio of SCFA producers and a low redox ratio;
Inoculum B: Inoculum with a low ratio of SCFA producers and a high redox ratio; as shown in Figure 8.

**Example 3: Determination of the optimal concentration for microbial inoculum administration**

An *in vivo* trial was performed to determine the inoculum concentration at which the animals do not lose performances. Animals were fed with a standard diet and different groups received concentrations between $10^5$ and $10^8$ CFU/animal of inocula.

Zootechnical performances were highly decreased when a high dose was given to the birds ($10^8$ CFU/bird) with a reduction of 10% of the body weight (BW) on the seventh day (D7) when compared with the non-inoculated control. In contrast, when animals received a dose of $10^{6-7}$ CFU/bird their BWs were similar to the non-inoculate group (see Figure 1).

In conclusion, the optimal microbial dose to be used for IMT and thus reduce animal stress and performance loses is $10^{6-7}$ CFU/bird.

**Example 4: Use of Inoculum A and Inoculum B of Example 2**

4.1) Situation 1: Non-challenging conditions

Animals were fed with a standard diet. The results are presented in Table 1 below.

Table 1: Effect of inocula considering the control group* (%)

|  | BW 14d | BWG 0-14d | ADG 0-14d | FI 0-14d | FCR 0-14d |
|---|---|---|---|---|---|
| STD + Inoculum A | 0.16 | 0.17 | 0.17 | 0.25 | -0.09 |
| STD + Inoculum B | -4.03 | -4.37 | -4.37 | -2.01 | 2.23 |
| * Control group = non-inoculated group fed with standard diet (STD) | | | | | |

From Table 1, it is shown that the group of chickens that received Inoculum A in non-challenging conditions didn't differ in terms of zootechnical parameters to non-inoculated group. In contrast, Inoculum B had a deleterious effect on the animals that received it, decreasing animals' body weight (BW) by 4% and increasing the Feed Conversion Ratio (FCR) by 2.23% which reflects on the higher feed intake (FI) of this group.

4.2) Situation 2: Nutritional challenge based on rye and pig fat starter and grower diet

Chickens were fed with a starter and grower diet based on wheat, rye and pig fat. Rye presents high amounts of Non-Starch Polysaccharides which increase digesta viscosity, decreasing motility of the small intestine and thereby producing a challenge condition (Challenge 1) into the bird.

The results are presented in Tables 2 and 3 below.

Table 2: Effect of inocula considering the challenge control group* on starter diet (%)

|  | BW 14d | BWG 0-14d | ADG 0-14d | FI 0-14d | FCR 0-14d |
|---|---|---|---|---|---|
| CHALLENGE 1 + Inoculum A | 4.69 | 5.13 | 5.13 | 3.55 | -1.54 |
| CHALLENGE 1 + Inoculum B | 3.01 | 3.29 | 3.29 | 6.22 | 2.76 |
| * Control group = non-inoculated group receiving challenge diet 1 | | | | | |

Administration of Inoculum A or Inoculum B to the birds following rye-pig fat dietary challenge increased their performance on day 14, being higher when the animals received the Inoculum A. This translates into +4.69% of BW, +5.13% in body weight gain (BWG) and a 1.54% decrease in FCR when Inoculum A is given. On the other hand, the Inoculum B resulted in a lower BW increase of 3%, and a big increase of FI (6.22%), what reduces the efficiency of the chicken production by 2.76% of FCR (Table2).

Table 3: Effect of inocula considering the challenge control group* on grower diet (%)

|  | BW 28d | BWG 14-28d | ADG 14-28d | FI 14-28d | FCR 14-28d |
|---|---|---|---|---|---|
| CHALLENGE 1 + Inoculum A | 3.34 | 2.74 | 2.73 | 4.51 | 2.08 |
| CHALLENGE 1 + Inoculum B | 0.61 | -0.46 | -0.46 | 1.90 | 2.78 |

(continued)

| | | BWG 0-28d | ADG 0-28d | FI 0-28d | FCR 0-28d |
|---|---|---|---|---|---|
| CHALLENGE 1 + Inoculum A | | 3.43 | 3.43 | 4.26 | 0.72 |
| CHALLENGE 1 + Inoculum B | | 0.62 | 0.62 | 3.01 | 2.17 |
| * Control group = non-inoculated group receiving challenge diet 1 | | | | | |

On day 28, only animals receiving Inoculum A showed an improvement on their performance by 3.34% BW while no improvement was recorded on the animals receiving Inoculum B (0.61%). Considering overall parameters, Inoculum A animals had a BWG of 3.43 instead of 0.62 with the Inoculum B. And those with the Inoculum A had a similar FCR (0.72%) to the non-inoculated rather than the increase of 2.17% of those administered with Inoculum B (Table 3).

4.3) Situation 3. Inflammatory and antinutritional grower diet (Galactomannan).

To create an anti-nutritional and inflammatory challenge (Challenge 2) in the animals, 2% of guar gum was added to the grower standard diet. Galactomannan creates a local inflammation in chickens increasing iNOS, K203 and IL-1 and a systemic effect on SAA, a1GP, IL-10 and IL-6 (Adisseo unpublished data) and decreases glucose absorption which in turn reduces animals' weight (Rainbird et al. 1984). It also increases digesta viscosity slowing intestinal movements translating to a greater opportunity for undesired microbial proliferation. Those microorganisms use the nutrients that could otherwise be used for the animal to grow.
The inocula were administered at day 0 and 14.
The results are presented in Table 4 below.

Table 4: Effect of inocula considering the grower challenge diet (%)

| | BW 28d | BWG 14-28d | ADG 14-28d | FI 14-28d | FCR 14-28d |
|---|---|---|---|---|---|
| CHALLENGE 2 + Inoculum A | 4,07 | 7,08 | 7,08 | 5,21 | -1,53 |
| CHALLENGE 2 + Inoculum B | 1,42 | 5,53 | 5,55 | 1,28 | -3,06 |
| | | BWG 0-28d | ADG 0-28d | FI 0-28d | FCR 0-28d |
| CHALLENGE 2 + Inoculum A | | 4,20 | 4,21 | 3,66 | -0,61 |
| CHALLENGE 2 + Inoculum B | | 1,46 | 1,46 | 0,21 | -1,21 |
| * Control group = non-inoculated group receiving challenge diet 2 | | | | | |

Animals receiving Inoculum A had better performances (+4.07% and +7.08% for BW and BWG) than those inoculated with Inoculum B (+1.42 and +5.53%) (Table 4).
In conclusion, it has been developed a method to perform IMT in chickens aiming to enhance animal resilience to challenging conditions. This method comprises (1) the administration of a microbial Inoculum at a concentration that ensures no loss of performance due to the inoculation: $10^7$ CFU/bird and (2) the characterization of the microbial inocula by the means of microbial ratios calculated from 16s sequencing data. Briefly, animals receiving a microbial Inoculum at $10^6$ to $10^7$ CFU/bird with a high SCFA producers' ratio and a low redox ratio will have lesser losses of performance (in terms of BW, BWG, FI and FCR) when confronted with a challenge in early life or a posteriori.

## Claims

1. A use of one or more ratios of quantities of bacteria in the microbiota of a farm animal, in animal feed, said ratio(s) being selected from *Firmicutes* / *Proteobacteria; Clostridium* Clusters IV and XIVa / *Enterobacteriaceae; Ruminococcaceae* and *Lachnospiraceae* / *Enterobacteriaceae;* butyrate-producing bacteria / *Firmicutes*; aerobic bacteria / anaerobic bacteria,

2. Use according to claim 1, as marker(s) to determine the status of the intestinal microbiota of a farm animal.

3. Use according to claim 1, for selecting an extract of a satisfactory intestinal microbiota of a farm animal, intended to maintain or to improve the intestinal microbiota of a different farm animal in need thereof.

4. A method for determining *in vitro* or ex *vivo* a status of the intestinal microbiota of a farm animal, comprising at least the following steps:

  detecting and quantifying, in a biological sample of said farm animal, the bacteria selected from the *Firmicutes*, *Proteobacteria, Clostridium* Clusters IV and XIVa, *Enterobacteria, Ruminococcaceae, Lachnospiraceae,* butyrate-producing bacteria, aerobic bacteria and anaerobic bacteria; and
  calculating at least one ratio selected from

  *Firmicutes* / *Proteobacteria,*
  *Clostridium* Clusters IV and XIVa / *Enterobacteriaceae,*
  *Ruminococcaceae* and *Lachnospiraceae* / *Enterobacteriaceae,*
  butyrate producing bacteria / *Firmicutes*, and
  aerobic bacteria / anaerobic bacteria.

5. Method according to claim 4, **characterized in that** it further comprises the following steps:

  comparing the or each of the ratios of said biological sample of said animal with a control value determined for each of said ratios, and
  determining the status of said microbiota from said comparison.

6. Method according to claim 5, **characterized in that** it comprises

  calculating at least one ratio selected from *Firmicutes* / *Proteobacteria, Clostridium* Clusters IV and XIVa / *Enterobacteriaceae, Ruminococcaceae* and *Lachnospiraceae* / *Enterobacteriaceae,* butyrate-producing bacteria / *Firmicutes*, and
  if the ratio or each of the calculated ratios is greater than or equal to the control value determined for the corresponding ratio, then the status of the microbiota is qualified as satisfactory, and if the ratio or each of the ratios is lower than the control value, then the status of the microbiota is qualified as unsatisfactory.

7. Method according to claim 5, **characterized in that** it comprises

  calculating at least the aerobic bacteria/anaerobic bacteria ratio, and
  if the ratio is less than or equal to the control value, then the status of the microbiota is qualified as satisfactory, and if the ratio is higher than the control value, then the status of the microbiota is qualified as unsatisfactory.

8. Method according to any one of claims 4-7, **characterized in that** it comprises calculating and optionally comparing with a control value, at least two, at least three, or at least four of said ratios, or the five ratios.

9. Method according to any one of claims 4 to 8, **characterized in that** the biological sample is selected from cecal content, ileal content and fecal content.

10. Method according to any one of the claims 4-9, **characterized in that** the quantity of bacteria is measured by the relative abundances obtained from data resulting from the sequencing analysis by 16s rRNA sequencing.

11. Method according to any one of claims 4-10, **characterized in that** the farm animal is a chicken and that the control value of any of the following ratios *Firmicutes* / *Proteobacteria;* Clostridium Clusters IV and XIVa / *Enterobacteriaceae; Ruminococcaceae* and Lachnospiraceae / *Enterobacteriaceae;* butyrate-producing bacteria / *Firmicutes*; aerobic bacteria / anaerobic bacteria is determined for a same chicken which is not.

12. Method according to any one of claims 4-11, **characterized in that** the farm animal is a chicken and that the control value of the aerobic bacteria / anaerobic bacteria ratio is measured in a chicken under farm conditions that that is not or less challenged than the chicken which intestinal microbiota status is determined.

13. Method for selecting an extract of a satisfactory intestinal microbiota of a farm animal, comprising the following steps:

  detecting and quantifying, in a biological sample of said farm animal, the quantity of each of the bacteria selected from *Firmicutes*, bacteria producing butyrate, aerobic bacteria and anaerobic bacteria and calculating at least one ratio selected from Butyrate-producing bacteria / *Firmicutes* (a1) and aerobic bacteria / anaerobic bacteria (a2);
detecting and quantifying, in a biological sample of a different farm animal, the quantity of each of the bacteria selected from *Firmicutes*, bacteria producing butyrate, aerobic bacteria and anaerobic bacteria and calculating at least one ratio selected from butyrate-producing bacteria / *Firmicutes* (b1) and aerobic bacteria / anaerobic bacteria (b2);
comparing the calculated ratios (a1) and (b1) and/or ratios (a2) and (b2); and
selecting said satisfactory extract from said comparison.

14. Method according to claim 13, **characterized in that**, if the butyrate-producing bacteria / *Firmicutes* ratio (a1) is greater than or equal to ratio (b1), and/or if the aerobic bacteria/anaerobic bacteria ratio (a2) is less than or equal to ratio (b2), said extract is selected.

15. Non-therapeutic method for maintaining or improving the intestinal microbiota of a farm animal in need thereof, **characterized in that** it comprises the following steps:

selecting a satisfactory microbiota extract according to the method of claim 13 or 14, and
administrating to said farm animal, said satisfactory microbiota extract or a derivative thereof resulting from fermentation.

16. Method according to claim 15, **characterized in that** the farm animal is young.

17. Method according to claim 16, **characterized in that** the farm animal is a chick and it is administered an amount of $1\text{-}9.10^7$ CFU of said extract.

18. A biomarker of the status of the microbiota of a farm animal selected from the ratios of amounts of bacteria, *Firmicutes* / *Proteobacteria; Clostridium* Clusters IV and XIVa / *Enterobacteriaceae; Ruminococcaceae* and *Lachnospiraceae* / *Enterobacteriaceae;* butyrate-producing bacteria / *Firmicutes*; aerobic bacteria / anaerobic bacteria.

FIG1. Ratio 2.1. *Firmicutes / Proteobacteria*

FIG 2. Ratio 2.3. *Clostridium* Cluster (CC)/ *Enterobacteriaceae*

FIG 3. Ratio 2.4. *Ruminococcaceae + Lachnospiraceae / Enterobacteriaceae*

FIG 4. Ratio 2.15. Butyrate producers / Firmicutes

FIG 5. Ratio 2.17. Overall butyrate producers / *Firmicutes*

FIG 6. Ratio Redox: Aerobic / Anaerobic bacteria

FIG 7. Ratio Ln Redox: Aerobic / Anaerobic bacteria

FIGURE 8

FIGURE 9

Europäisches
Patentamt

European
Patent Office

Office européen
des brevets

# EUROPEAN SEARCH REPORT

**Application Number**

EP 23 18 2883

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | COSTA MARCIO C. ET AL: "Different antibiotic growth promoters induce specific changes in the cecal microbiota membership of broiler chicken", PLOS ONE, vol. 12, no. 2, 1 January 2017 (2017-01-01), page e0171642, XP093059867, DOI: 10.1371/journal.pone.0171642 Retrieved from the Internet: URL:https://journals.plos.org/plosone/article/file?id=10.1371/journal.pone.0171642&type=printable> | 1,2, 4-12,18 | INV. C12Q1/689 |
| Y | * the whole document * | 3 | |
| X | CASTILLO ET AL: "Adaptation of gut microbiota to corn physical structure and different types of dietary fibre", LIVESTOCK SCIENCE, ELSEVIER, AMSTERDAM, NL, vol. 109, no. 1-3, 29 April 2007 (2007-04-29), pages 149-152, XP022053293, ISSN: 1871-1413, DOI: 10.1016/J.LIVSCI.2007.01.129 * the whole document * | 1 | |
| Y | WO 2021/001642 A1 (OCEAN HARVEST TECH UK [GB]) 7 January 2021 (2021-01-07) * the whole document * | 13-17 | |
| Y | US 2016/015757 A1 (MIZRAHI ITZHAK [IL] ET AL) 21 January 2016 (2016-01-21) * the whole document * | 3,13-17 | |

**TECHNICAL FIELDS SEARCHED (IPC)**

C12Q

The present search report has been drawn up for all claims

| Place of search | Date of completion of the search | Examiner |
|---|---|---|
| The Hague | 29 November 2023 | Cornelis, Karen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 18 2883

This annex lists the patent family members relating to the patent documents cited in the above-mentioned European search report.
The members are as contained in the European Patent Office EDP file on
The European Patent Office is in no way liable for these particulars which are merely given for the purpose of information.

29-11-2023

| Patent document cited in search report | Publication date | Patent family member(s) | Publication date |
|---|---|---|---|
| WO 2021001642 A1 | 07-01-2021 | EP 3989732 A1 | 04-05-2022 |
| | | GB 2594433 A | 03-11-2021 |
| | | US 2022322702 A1 | 13-10-2022 |
| | | WO 2021001642 A1 | 07-01-2021 |
| US 2016015757 A1 | 21-01-2016 | US 2016015757 A1 | 21-01-2016 |
| | | WO 2014141274 A1 | 18-09-2014 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DUCATELLE, R.** ; **GOOSSENS, E.** ; **DE MEYER, F.** ; **EECKHAUT, V.** ; **ANTONISSEN, G.** ; **HAESEB-ROUCK, F.** ; **VAN IMMERSEEL, F.** Biomarkers for monitoring intestinal health in poultry. Present status and future perspectives. *Veterinary research*, 2018, vol. 49 (1), 43
- **EICHNER, G.** ; **VIEIRA, S. L.** ; **TORRES, C. A.** ; **CONEGLIAN, J.L.B.** ; **FREITAS, D. M.** ; **OYARZA-BAL, O. A.** Litter Moisture and Footpad Dermatitis as Affected by Diets Formulated on an All-Vegetable Basis or Having the Inclusion of Poultry By-Product. *The Journal of Applied Poultry Research*, 2007, vol. 16 (3), 344-350

- **WGS ST PARK** ; **J. KIM**. Trends in Next-Generation Sequencing and a New Era for Whole Genome Sequencing. *Int. Neurourol J.*, November 2016, vol. 20 (2), S76-83
- **VITAL, M.** ; **KARCH, A.** ; **PIEPER, D. H.** Colonic Butyrate-Producing Communities in Humans: an Overview Using Omics Data. *mSystems*, 2017, vol. 2 (6)
- **MILLION, M.** ; **RAOULT, D.** Linking gut redox to human microbiome. *Human Microbiome Journal*, 2018, vol. 10, 27-32